(19) Europäisches Patentamt
European Patent Office
Office européen des brevets

(11) Publication number: **0 152 542**
**A1**

# (12) EUROPEAN PATENT APPLICATION

(21) Application number: **84113398.6**

(22) Date of filing: **07.11.84**

(51) Int. Cl.[4]: **C 07 D 207/34**, A 61 K 31/40

(30) Priority: **14.11.83 IT 2369883**

(43) Date of publication of application: **28.08.85 Bulletin 85/35**

(84) Designated Contracting States: **AT BE CH DE FR GB IT LI LU NL SE**

(71) Applicant: **POLI INDUSTRIA CHIMICA S.p.A., Piazza Agrippa, 1, I-20141 Milano (IT)**

(72) Inventor: **Massaroli, Gian Giacomo, Via Lauro, Rovagnate Como (IT)**

(74) Representative: **Bianchetti, Giuseppe, Studio Consulenza Brevettuale Via Senato 24, I-20121 Milan (IT)**

(54) **Pyrrol-3-carboxylic acids having central analgesic activity.**

(57) Compounds of general formula (I)

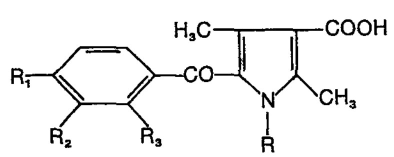

(I)

wherein

R is $CH_3$, n-$C_4H_9$, benzyl or o-Cl-benzyl
$R_1$ is Cl, $CH_3$, $OCH_3$
$R_2$ is H or Cl
$R_3$ is H or Cl

(with the proviso that when R = $CH_3$ and $R_2$ = $R_3$ = H, $R_1$ is different from chlorine)
and the corresponding salts with inorganic cations or deriving from non-toxic, pharmaceutically acceptable organic bases, such as primary, secondary or tertiary amines or aminoacids, are described.

A process for the preparation thereof and pharmaceutical compositions therefrom having analgesic activity are moreover described.

EP 0 152 542 A1

"Pyrrol-3-carboxylic acids having central analgesic activity"

The present invention refers to compounds having the formula:

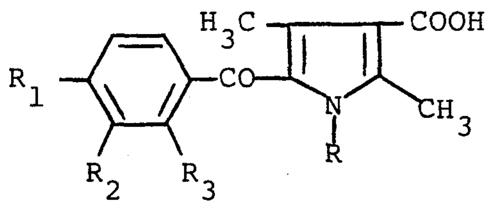

(I)

wherein

R is $CH_3$, n-$C_4H_9$, benzyl or o-Cl-benzyl

$R_1$ is Cl, $CH_3$, $OCH_3$

$R_2$ is H or Cl

$R_3$ is H or Cl

(with the proviso that when R = $CH_3$ and $R_2$ = $R_3$ = H, $R_1$ is different from chlorine)

and the corresponding salts with inorganic cations or deriving from non-toxic, pharmaceutically acceptable organic bases, such as primary, secondary or tertiary amines or aminoacids.

Another object of the invention is provided by a process for the preparation of compounds of formula I.

Finally, the invention concerns pharmaceutical compositions having analgesic action made up by, or comprising as the active principle, at least one of the compounds of formula I.

Said compounds are, in fact, endowed with an high analgesic activity of the central type while being

devoid of addiction side-effects, so frequent in this class of analgesics.

Compounds having a similar structure to (I) and active as analgesics or antiinflammatories, are known. For instance, German Patent No. 2,102,746 claims the 5-(p-chlorobenzoyl)-1,4-dimethyl-pyrrol-2-acetic acid (Zomepirac) and European patent applications No. 32314 and 72013 disclose 5-aroyl-pyrrol-2-acetic acids differently substituted in positions 1 and/or 4, but they are also devoid of substituents in position 3. Zomepirac is a typical peripheral analgesic which, differently from compounds I, inhibits the cyclooxygenase and the prostaglandinesynthetase.

As far as the Zomepirac derivatives described in European patent applications No. 23314 and 72013 are concerned, no test is given showing a central effect and such compounds are particularly defined as "antiinflammatory agents" and "Zomepirac type compounds".

The central analgesic action of compounds (I), which are chemically different from the known similar aroylpyrrole derivatives mainly because of the lack of acidic groups in the chain in position 2 and the presence of the carboxy group in position 3, must be therefore considered as surprising.

According to the present invention, the compounds of formula I can be conveniently prepared according to the following reaction scheme:

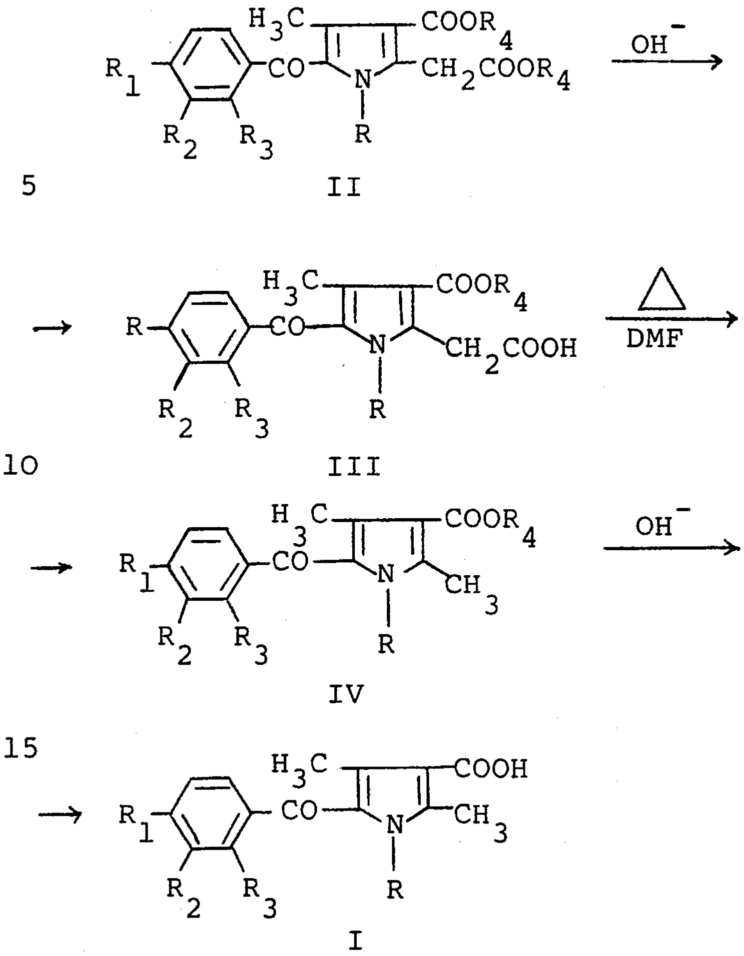


II

III

IV

I

wherein $R_4$ represents a $C_1$-$C_4$ alkyl residue.

The hydrolysis of the diester II with equimolar amounts of sodium hydroxyde in aqueous isopropanol occurs selectively and in good yields at the level of the acetic carboxy group linked in position 2 of the pyrrole nucleus.

The reaction course is unequivocally confirmed by the NMR spectrum of compounds I, which clearly shows the presence of a methyl group in position 2 of the pyrrole ring.

The following examples illustrate the invention

without limiting it in any way.

EXAMPLE 1

1,4-Dimethyl-5-(3,4-dichlorobenzoyl)-3-ethoxycarbonyl-pyrrol-2-acetic acid (III, R = $CH_3$; $R_1$, $R_2$ = Cl; $R_3$ = H; $R_4$ = $C_2H_5$)

42.6 Grams (0.1 moles) of ethyl-1,4-dimethyl-5-(3,4-dichlorobenzoyl)-3-ethoxycarbonyl-pyrrol-2-acetate, are suspended in 300 ml of an isopropanol-water (80:20) mixture, which is heated to reflux. 100 Ml of 1N sodium hydroxide solution are then added dropwise, in 30 minutes; after 2 hours at reflux, the solution is cooled and the isopropanol is evaporated under reduced pressure.

By acidification with hydrochloric acid to pH 3 a precipitate is obtained, which is filtered, washed with water, dried and crystallized from ethyl acetate, to give 25.5 g (75% yield) of a product having melting point 156-158°C. In a similar procedure, the products reported in Table 1 are obtained.

TABLE 1

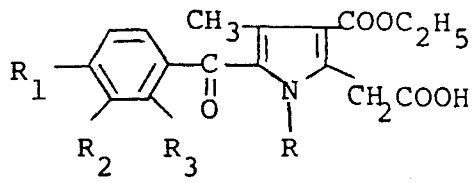

| R | $R^1$ | $R^2$ | $R^3$ | Elementary formula | M.p. |
|---|---|---|---|---|---|
| $CH_3$ | $CH_3$ | H | H | $C_{19}H_{21}NO_5$ | 125-127° |
| $CH_3$ | $OCH_3$ | H | H | $C_{19}H_{21}NO_6$ | 178-180° |
| $CH_3$ | Cl | H | H | $C_{18}H_{18}ClNO_5$ | 156-157° |
| n-$C_4H_9$ | Cl | H | H | $C_{21}H_{24}ClNO_5$ | 174-176° |
| $CH_3$ | Cl | H | Cl | $C_{18}H_{17}Cl_2NO_5$ | 123-125° |
| 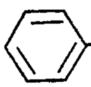-$CH_2$ | Cl | H | H | $C_{24}H_{22}ClNO_5$ | 110-112° |
| (*) 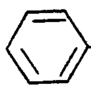-$CH_2$ | Cl | H | H | $C_{24}H_{21}Cl_2NO_5$ | 156-158° |

(*) Methyl ester

EXAMPLE 2

1,2,4-Trimethyl-5-(3,4-dichlorobenzoyl)-pyrrol-3-ethyl carboxylate (IV; R = $CH_3$; $R_1$, $R_2$ = Cl; $R_3$ = H; $R_4$ = $C_2H_5$)

39.8 Grams of 1,4-dimethyl-5-(3,4-dichlorobenzoyl)-3-ethoxycarbonylpyrrol-2-acetic acid (0.1 moles) are dissolved in 120 ml of N,N-dimethylformamide and the solution is refluxed for 2 hours in the presence of 400 mg of reduced copper. The mixture is finally cooled and the solvent evaporated under vacuum. The evaporation residue is treated with 100 ml of water and 100 ml of ethyl acetate. The mixture is filtered on celite to remove copper.

The aqueous phase is separated and the organic phase is washed with a sodium bicarbonate solution, dried on sodium sulphate, filtered and evaporated under vacuum to small volume. After dilution with petroleum ether, the crystallization product is obtained. There were obtained 29.4 g, yield 83%, m.p. 128-128°C.

By a similar procedure the compounds reported in Table 2 are obtained.

TABLE 2

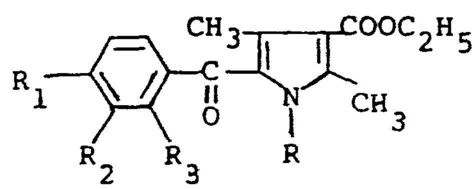

| R | $R^1$ | $R^2$ | $R^3$ | Elementary formula | M.p. |
|---|---|---|---|---|---|
| $CH_3$ | $CH_3$ | H | H | $C_{18}H_{21}NO_3$ | 105-107° |
| $CH_3$ | $OCH_3$ | H | H | $C_{18}H_{21}NO_4$ | 102-104° |
| $CH_3$ | Cl | H | H | $C_{17}H_{18}ClNO_3$ | 120-122° |
| $n$-$C_4H_9$ | Cl | H | H | $C_{20}H_{24}ClNO_3$ | oil |
| $CH_3$ | Cl | H | Cl | $C_{17}H_{17}Cl_2NO_3$ | 120-121° |
| 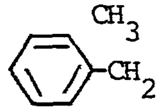 | Cl | H | H | $C_{23}H_{22}ClNO_3$ | oil |
| (*) 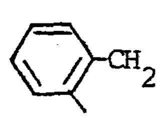 | Cl | H | H | $C_{23}H_{21}Cl_2NO_3$ | oil |

(*) Methyl ester

EXAMPLE 3

1,2,4-Trimethyl-5-(2,4-dichlorobenzoyl)-pyrrol-3-carboxylic acid (I, $R = CH_3$; $R_1$, $R_3$ = Cl; $R_2$ = H)

35.4 Grams of 1,2,4-trimethyl-5-(2,4-dichloro-

benzoyl)-pyrrol-3-ethyl carboxylate (0.1 moles) are refluxed for 3 hours in a mixture of 100 ml of 15% aqueous sodium hydroxide and 100 ml of ethanol. The mixture is finally cooled, the ethanol is evaporated at reduced pressure, the residue is diluted with 200 ml of water and acidified with hydrochloric acid, obtaining a precipitate which is filtered, washed with water and crystallized from ethanol, obtaining 28.3 g (83% yield) m.p. 218-219°.

By a similar procedure, the products reported in Table 3 are obtained.

TABLE 3

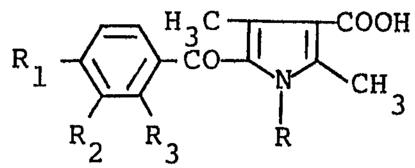

| R | $R_1$ | $R_2$ | $R_3$ | FORMULA | M.P. | $LD_{50}$ MICE OS | $LD_{50}$ RAT OS | PHENYLQUINONE $ED_{50}$ OS | HOT PLATE $ED_{50}$ S.C. MICE |
|---|---|---|---|---|---|---|---|---|---|
| $CH_3$ | $CH_3$ | H | H | $C_{16}H_{17}NO_3$ | 223-225° | 1400 | >1600 | 10 | > 10 |
| $CH_3$ | $OCH_3$ | H | H | $C_{16}H_{17}NO_4$ | 199-201° | >1600 | >1600 | 8.4 | 7.6 |
| *$CH_3$ | Cl | H | H | $C_{15}H_{14}ClNO_3$ | 210-212° | 800 | >1600 | 4.6 | 2.3 |
| $C_4H_9$ | Cl | H | H | $C_{18}H_{20}ClNO_3$ | 205-206° | 1300 | >1600 | 6.3 | 2.9 |
| $CH_3$ | Cl | H | Cl | $C_{15}H_{13}Cl_2NO_3$ | 218-219° | 1400 | 1700 | 10.9 | 5.7 |
| $CH_3$ | Cl | Cl | H | $C_{15}H_{13}Cl_2NO_3$ | 208-210° | 800 | >1600 | 7.5 | 4.7 |
| 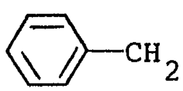 | Cl | H | H | $C_{21}H_{18}ClNO_3$ | 225-227° | 1500 | >1600 | 7.6 | 4.8 |
| 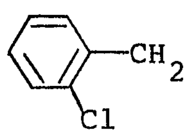 | Cl | H | H | $C_{21}H_{17}Cl_2NO_3$ | 208-210° | 1200 | >1600 | 10.8 | 5.5 |

(*) known from Japan 57-2268, which however does not report any pharmacological property.

The pharmaco-toxicological properties of the reference compounds will be hereinafter reported.

The analgesic activity of the compounds of formula I, administered by the oral route has been determined by means of the following tests:

- Phenylquinone test in mice (Hendershot and Forsaith - J. Pharmacol. Exp. Ther. 123, 237 (1959))
- Hot plate test - Male albino mice, treated with increasing doses of the compound under test, are placed on a thermostatized plate at 55°C. The time necessary for the mouse to react to the painful stimulus is then measured. An effective dose 50 ($ED_{50}$) is defined as the dose inducing a 50% increase of the reaction time, with respect to control animals.
- The acute toxicity per os has been studied in the mouse and in the rat and expressed as $LD_{50}$ at the 14th observation day.

The results of the above tests are reported in Table 3.

The doses shown in the Table are in mg/kg per os.

As a comparison codeine hydrochloride is used, which in said tests gave the following results:

$LD_{50}$ mouse per os : 300 mg/kg
$ED_{50}$ phenylquinone : 5.6 mg/kg
$ED_{50}$ hot plate : 25 mg/kg

The remarkable activity of the compounds of formula I in the hot plate test shows that the analgesic activity of said compounds is of the central type.

The compounds of formula I, differently from codeine hydrochloride, do not induce the phenomenon of "Jumping by Nalorphine" in the mouse, which excludes that said compounds could induce physical addiction.

The compounds of formula I are moreover unequivocally different from known compounds having similar structure, i.e. such as Zomepirac, because they are endowed with central analgesic activity and devoid of activity inhibiting the cyclooxygenase and the prostaglandinsynthetase. As a result, the compounds of formula I are devoid of ulcerogenic activity and do not cause the formation of leukotrienes, responsible of the anaphylattoid reactions so common with Zomepirac and with other compounds having analgesic-antiinflammatory activities.

The present invention refers also to all the industrially applicable aspects connected with the use of compounds of formula I as therapeutic agents. An essential aspect of the invention is therefore provided by pharmaceutical composition containing, as the active principle, predetermined and therapeutically effective amounts of at least one of the compounds of formula I or salts thereof, in addition to optional excipients of conventional use in pharmaceutical technique, useful for the treatment of acute and chronic pain deriving from neoplasias, surgical operations, headache, toothache.

The compounds of the present invention can be administered by the oral, rectal or parenteral route, for instance in form of capsules, tablets, sugar-coated tablets, syrups, drops, suppositories, microclisms, ampoules.

The following formulations are cited as an example:

a) tablets containing from 25 to 500 mg of a compound of formula I, with excipients and disgregating agents commonly used in pharmaceutical technique;

b) suppositories containing from 50 to 1000 mg of a compound of formula I;

c) ampoules for intramuscular or endovenous use containing from 25 to 1000 mg of a compound of formula I, in forma of a salt thereof, having a pH around physiological values.

CLAIMS for the Contracting States:

BE, CH, FR, DE, GB, IT, LI, LU, NL, SE

1. Compounds of general formula (I)

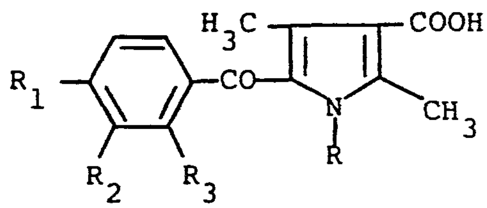

(I)

wherein

R is $CH_3$, n-$C_4H_9$, benzyl or o-Cl-benzyl

$R_1$ is Cl, $CH_3$, $OCH_3$

$R_2$ is H or Cl

$R_3$ is H or Cl

(with the proviso that when R = $CH_3$ and $R_2$ = $R_3$ = H, $R_1$ is different from chlorine) and the corresponding salts with inorganic cations or deriving from non-toxic, pharmaceutically acceptable organic bases, such as primary, secondary or tertiary amines or aminoacids.

2. A compound according to claim 1 selected in the group consisting of: 1,2,4-trimethyl-5-(2,4-dichlorobenzoyl)-pyrrol-3-carboxylic acid; 1,2,4-trimethyl-5-(3,4-dichloro-benzoyl)-pyrrol-3-carboxylic acid; 1,2,4-trimethyl-5-(4-methyl-benzoyl)-pyrrol-3-carboxylic acid; 1,2,4-trimethyl-5-(4-methoxy-benzoyl)-pyrrol-3-carboxylic acid; 1-n-butyl-2,4-dimethyl-5-(4-chlorobenzoyl)-pyrrol-3-carboxylic acid; 1-benzyl-2,4-dimethyl-5-(4-chlorobenzoyl)pyrrol-3-carboxylic acid;

1-(2-chlorobenzyl)-2,4-dimethyl-5-(4-chlorobenzoyl)-pyrrol-3-carboxylic acid
and salts thereof.

3. 1,2,4-Trimethyl-5-(2,4-dichlorobenzoyl)-pyrrol-3-carboxylic acid and salts thereof.

4. Process for the preparation of compounds of claims 1-8, characterized by comprising the following steps:

a) selective hydrolysis of a diester of formula I to give an acid ester of formula III;
b) decarboxylation of an acid-ester of formula III to give an ester of formula IV;
c) hydrolysis of an ester of formula IV to give a final product of formula I, according to the following scheme:

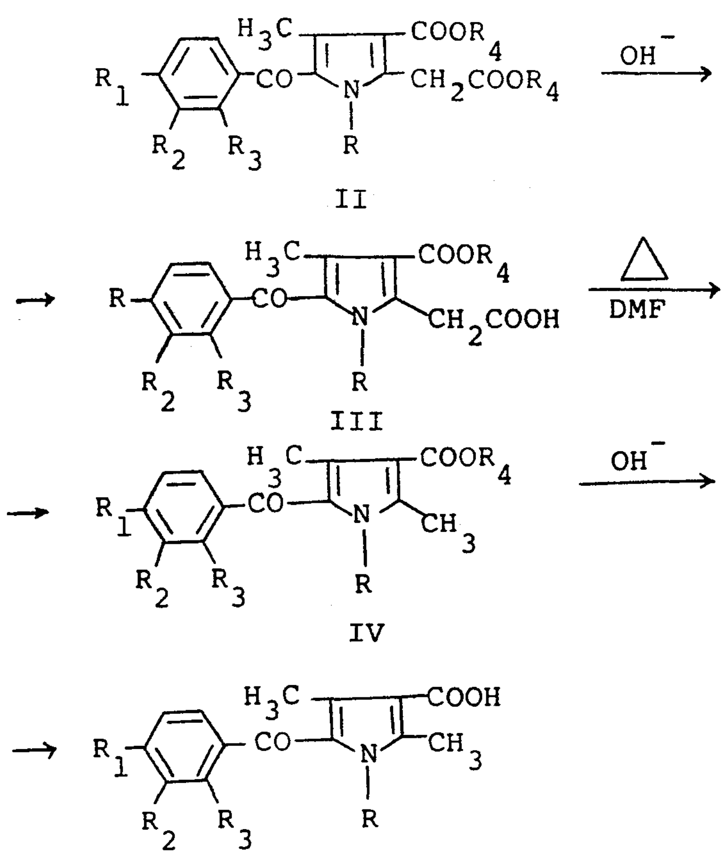


II

III

IV

I

wherein R, $R_1$, $R_2$, $R_3$ have the above precised meanings, and $R_4$represents a $C_1$-$C_4$ alkyl residue.

5. Process according to claim 4, characterized in that the hydrolysis of the diester of formula II is carried out with equimolar amounts of an alkali metal hydroxide in hydroalcoholic solution.

6. Process according to claims 4 or 5 characterized in that the hydrolysis of the diester of formula II is carried out with equimolar amounts of sodium hydroxide in aqueous isopropanol.

7. Pharmaceutical compositions having central analgesic activity characterized by containing as an active principle one or more compounds of formula I wherein:

R is $CH_3$, n-$C_4H_9$, benzyl or o-chloro-benzyl

$R_1$ is Cl, $CH_3$, $OCH_3$

$R_2$ is H or Cl

$R_3$ is H or Cl

and the respective salts with inorganic cations or deriving from non-toxic, pharmaceutically acceptable, organic bases such as primary, secondary, tertiary amines of aminoacids.

8. Pharmaceutical compositions according to claim 7 characterized by containing as the active principle at least a compound selected in the group consisting of

1,2,4-trimethyl-5-(2,4-dichlorobenzoyl)-pyrrol-3-carboxylic acid; 1,2,4-trimethyl-5-(3,4-dichlorobenzoyl)-pyrrol-3-carboxylic acid; 1,2,4-trimethyl-5-(4-methyl-benzoyl)-pyrrol-3-carboxylic acid;

1,2,4-trimethyl-5-(4-methoxy-benzoyl)-pyrrol-3-carboxylic acid; 1-n-butyl-2,4-dimethyl-5-(4-chlorobenzoyl)-pyrrol-3-carboxylic acid; 1-benzyl-2,4-dimethyl-5-(4-chlorobenzoyl)-pyrrol-3-carboxylic acid; 1-(2-chlorobenzyl)-2,4-dimethyl-5-(4-chlorobenzoyl)-pyrrol-3-carboxylic acid; 1,2,4-trimethyl-5-(4-chlorobenzoyl)-pyrrol-3-carboxylic acid.

9. Pharmaceutical compositions according to claims 7 or 8, to be administered by the oral route, in unit doses from 25 to 500 mg of active principle.

10. Pharmaceutical compositions according to claims 7 or 8, to be administered by the rectal route, in unit doses from 50 to 1000 mg of active principle.

11. Pharmaceutical compositions according to claims 7 or 8, to be administered by the parenteral route, in unit doses from 25 to 500 mg of active principle.

CLAIMS for AT

1. Process for the preparation of compounds of general formula (I)

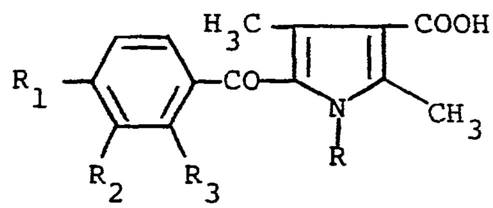

(I)

wherein

R is $CH_3$, $n-C_4H_9$, benzyl or o-Cl-benzyl

$R_1$ is Cl, $CH_3$, $OCH_3$

$R_2$ is H or Cl

$R_3$ is H or Cl

(with the proviso that when R = $CH_3$ and $R_2 = R_3$ = H, $R_1$ is different from chlorine)

and the corresponding salts with inorganic cations or deriving from non-toxic, pharmaceutically accep table organic bases, such as primary, secondary or tertiary amines or aminoacids,

characterized by comprising the following steps:

a) selective hydrolysis of a diester of formula I, to give and acid ester of formula III;

b) decarboxylation of an acid-ester of formula III to give an ester of formula IV;

c) hydrolysis of an ester of formula IV to give a final product of formula I, according to the following scheme:

===

===

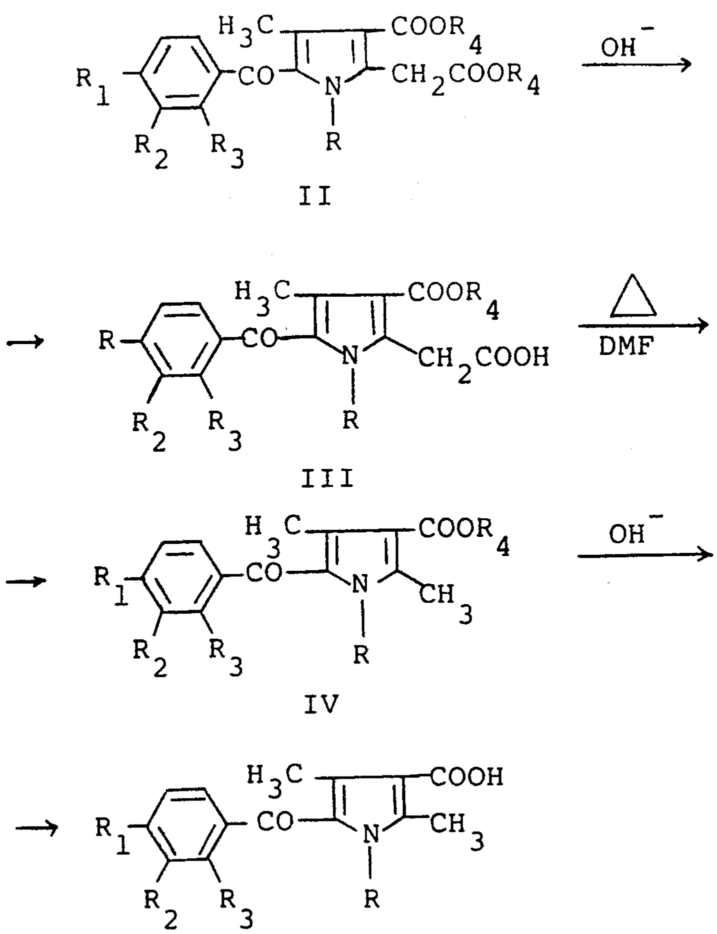


II

III

IV

I

wherein R, $R_1$, $R_2$, $R_3$ have the above precised meanings, and $R_4$ represents a $C_1$-$C_4$ alkyl residue.

2. Process according to claim 1, characterized in that the hydrolysis of the diester of formula II is carried out with equimolar amounts of an alkali metal hydroxide in hydroalcoholic solution.

3. Process according to claims 1 or 2 characterized in that the hydrolysis of the diester of formula II is carried out with equimolar amounts of sodium hydroxide in aqueous isopropanol.

European Patent Office

**EUROPEAN SEARCH REPORT**

0152542

Application number

EP 84 11 3398

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | FR-A-2 405 936 (ALBERT ROLLAND)<br>* Whole document *<br>--- | 1-3, 7-11 | C 07 D 207/34<br>A 61 K 31/40 |
| X | US-A-4 282 242 (G.F. HOLLAND)<br>* Abstract; column 2, figure IV; Columns 38-41 *<br>----- | 1-3, 7-11 | |
| | | | TECHNICAL FIELDS SEARCHED (Int. Cl.4) |
| | | | C 07 D 207/00 |

The present search report has been drawn up for all claims

| Place of search | Date of completion of the search | Examiner |
|---|---|---|
| THE HAGUE | 04-03-1985 | MAISONNEUVE J. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503. 03.82